# EUROPÄISCHE PATENTANMELDUNG

(11) **EP 4 074 346 A1**
(43) Veröffentlichungstag der Anmeldung: **19.10.2022**
(21) Anmeldenummer: 22166853.6
(22) Anmeldetag: 06.04.2022
(51) Int. Cl.: A61L 2/00, B08B 9/00, B01L 3/00

(54) **VERBINDUNGSEINHEIT ZUM VERBINDEN EINES VERBINDUNGSELEMENTES UND VERFAHREN ZUM HERSTELLEN EINER VERBINDUNGSEINHEIT, REINIGUNGSGERÄT**

(30) Priorität: 16.04.2021 DE 102021109627
(71) Anmelder: Miele & Cie. KG, 33332 Gütersloh (DE)
(72) Erfinder: Neufeld, Roman, 33619 Bielefeld (DE)

(57) **Zusammenfassung**

Die Erfindung betrifft eine Verbindungseinheit (100) zum Verbinden eines Verbindungselementes (110) mit einem Rohrabschnitt (115) für einen Einsatz in einem Reinigungsgerät. Die Verbindungseinheit (100) weist ein Rohr (102) mit dem Rohrabschnitt (115) auf, wobei der Rohrabschnitt (115) eine Durchgangsöffnung (140) aufweist, die ausgebildet ist, um zumindest einen Teil eines Verbindungselementes (110) aufzunehmen. Die Verbindungseinheit (100) weist ferner das Verbindungselement (110) mit einem Gewindeabschnitt (120) und einem Kontaktabschnitt (125) auf, wobei der Gewindeabschnitt (120) ausgebildet ist, um ein Fixierelement (105) aufzunehmen, und wobei der Kontaktabschnitt (125) ausgebildet ist, um den zumindest einen Teil des Verbindungselementes (110) im Rohrabschnitt (115) formschlüssig mit dem Rohr (102) zu verbinden.

## Beschreibung

Die Erfindung betrifft eine Verbindungseinheit zum Verbinden eines Verbindungselementes, ein Verfahren zum Herstellen einer Verbindungseinheit und ein Reinigungsgerät.

Für die Innenreinigung von z. B. Laborglas oder medizinischem Spülgut werden Düsen und diverse Adapter verwendet. Um diese mit den Rohren der Körbe zu verbinden, welche die Spülflotte für die Innenreinigung führen, sollte eine Verbindung geschaffen werden, die wirtschaftlich herstellbar, mechanisch robust und wieder lösbar ist. Sowohl bei der Anmelderin als auch bei Mitbewerbern werden Gewindebuchsen/ Schweißmuttern eingesetzt, die auf das Rohr geschweißt werden. Neben den Schweißmuttern können Gewinde durch Gewindeschneiden oder Gewindeformen hergestellt werden.

Der Erfindung stellt sich die Aufgabe eine verbesserte Verbindungseinheit zum Verbinden eines Verbindungselementes, ein verbessertes Verfahren zum Herstellen einer Verbindungseinheit und ein verbessertes Reinigungsgerät zu schaffen.

Erfindungsgemäß wird diese Aufgabe durch eine Verbindungseinheit zum Verbinden eines Verbindungselementes, durch ein Verfahren zum Herstellen einer Verbindungseinheit und durch ein Reinigungsgerät mit den Merkmalen der Hauptansprüche gelöst. Vorteilhafte Ausgestaltungen und Weiterbildungen der Erfindung ergeben sich aus den nachfolgenden Unteransprüchen.

Die mit der Erfindung erreichbaren Vorteile bestehen darin, dass eine Verbindungseinheit geschaffen wird, die eine einfache Montage und Demontage von einem Düsenelement gewährleistet.

Eine Verbindungseinheit zum Verbinden eines Verbindungselementes mit einem Rohrabschnitt für einen Einsatz in einem Reinigungsgerät, weist die folgenden Merkmale auf:
ein Rohr mit dem Rohrabschnitt, wobei der Rohrabschnitt eine Durchgangsöffnung aufweist, die ausgebildet ist, um zumindest einen Teil eines Verbindungselementes aufzunehmen; und
das Verbindungselement mit einen Gewindeabschnitt und einem Kontaktabschnitt, wobei der Gewindeabschnitt ausgebildet ist, um ein Fixierelement aufzunehmen, und wobei der Kontaktabschnitt ausgebildet ist, um den zumindest einen Teil des Verbindungselementes im Rohrabschnitt formschlüssig mit dem Rohr zu verbinden.

Unter einem Verbindungselement kann ein Element mit einem Gewinde verstanden werden, welches ein Innengewinde und/oder ein Außengewinde aufweist. Das Außengewinde ist dabei ausgebildet, um ein Fixierelement aufzunehmen. Bei dem Fixierelement kann es sich um eine Mutter handeln, beispielsweise eine Sechskantmutter. Zusätzlich weist das Verbindungselement einen Kontaktabschnitt auf. Unter einem Kontaktabschnitt kann eine beispielsweise viereckige Platte verstanden werden, die ausgebildet ist, um das Verbindungselement formschlüssig mit dem Rohrabschnitt des Rohres zu verbinden. Bei dem Rohr kann es sich um ein Rohr handeln, durch welches ein Reinigungsfluid geleitet wird. Der Rohrabschnitt des Rohres weist eine Durchgangsöffnung auf, in die das Verbindungselement gekoppelt wird und durch die das Reinigungsfluid geleitet wird. Das Rohr kann beispielsweise Bestandteil eines Korbes zum Reinigen von Reinigungsgut sein oder für oder als ein solcher verwendet werden. Der Korb kann sich in einem Reinigungsgerät befinden, beispielsweise einem medizinischen Reinigungsgerät zum Reinigen von medizinischem Reinigungsgut, wie beispielsweise Laborglas.

Der hier vorgestellte Ansatz ermöglicht die Realisierung einiger Vorteile. Der Fertigungsaufwand bei der Verwendung von erfindungsgemäßen Verbindungselementen bzw. Verbindungseinheiten ist beispielsweise gering, da ein aufwendiger Schweißvorgang entfällt und die Rohre aufgrund des entfallenden Schweißverzugs nicht gerichtet werden brauchen. Eine anschließende Oberflächenbehandlung (beizen und passivieren) ist beispielsweise nicht mehr notwendig. Es entstehen keine Späne die entfernt werden sollten, da diese gerade im medizinischen Bereich kritisch sind. Die Gewindetiefe ist unabhängig von der Wandstärke des Rohres. Das Gewinde ist sehr tragfähig und kann wegen der hohen/dicken Wandstärke nicht leicht ausreißen. Die Gewindetiefe ist optimal, sodass der Düsenanschluss nicht in das Rohr hineinragt, was den Strömungsfluss nicht beeinträchtigt. Allen genannten Möglichkeiten zur Erstellung eines Gewindes ist der Vorteil gemeinsam, dass nach Fertigstellung des Gewindeanschlusses das Gewinde geändert oder getauscht werden kann. Wird beispielsweise für eine größere Düse ein anderes Gewinde benötigt, ist es nachträglich möglich das Gewinde einfach zu ändern. Durch die Verwendung des hier vorgestellten Ansatzes kann das bisherige Schweißen, der damit verbundene Verzug und das Richten, sowie die Oberflächenbehandlung entfallen. Der Gewindeeinsatz kann im Zuge der ohnehin erforderlichen Düsenmontage erfolgen und benötigt keine Spezialwerkzeuge. Diese Vorteile führen zu Einsparungen der Fertigungs-/ und gegebenenfalls Lagerhaltungskosten. Die einfache Montage und Demontage ermöglichen auch einen Austausch im Fehlerfall, der vorher nicht möglich war. Dies führt zu einer höheren Kundenzufriedenheit.

Der Kontaktabschnitt kann gemäß einer Ausführungsform im Querschnitt eine Form aufweisen, die einer Form der Durchgangsöffnung entspricht, um den zumindest einen Teil des Verbindungselementes durch die Durchgangsöffnung aufzunehmen. Dies bietet den Vorteil, dass die Durchgangsöffnung den Kontaktabschnitt aufnehmen kann. Der Bediener kann den Kontaktabschnitt ohne großen Aufwand in der Durchgangsöffnung montieren und demontieren. Dies erhöht die Zufriedenheit des Bedieners.

Die Durchgangsöffnung im Rohrabschnitt und/oder der Kontaktabschnitt kann gemäß einer Ausführungsform mehreckig insbesondere im Wesentlichen dreieckig oder viereckig ausgeformt sein. Dies bietet den Vorteil, dass die Durchgangsöffnung und der Kontaktabschnitt leichter hergestellt werden können, da die Form beispielsweise ausgestanzt werden kann.

Der Kontaktabschnitt in dem Rohrabschnitt kann gemäß einer Ausführungsform verkantbar ausgebildet sein. Das bedeutet, dass die größte Quererstreckung vom Kontaktabschnitt größer ist als die Breite vom Rohrabschnitt. Dadurch ist der Kontaktabschnitt verkantbar, was einen optimalen Sitz des Kontaktabschnittes des Verbindungselementes in dem Rohrabschnitt gewährleistet.

Bei der Verbindungseinheit kann gemäß einer Ausführungsform das Verbindungselement einen Halsabschnitt zwischen dem Kontaktabschnitt und dem Gewindeabschnitt aufweisen, wobei der Halsabschnitt eine Länge aufweist, die mindestens einer Dicke einer Wand des Rohres im Rohrabschnitt entspricht. Dadurch ist ein optimaler Sitz des Verbindungselementes an dem Rohrabschnitt gewährleistet.

Der Halsabschnitt kann gemäß einer Ausführungsform einen Querschnitt aufweisen, der höchstens einer Breite des Kontaktabschnittes entspricht. Dadurch ist ein optimaler Sitz des Verbindungselementes an dem Rohrabschnitt gewährleistet.

Das Verbindungselement kann gemäß einer Ausführungsform im Gewindeabschnitt ein Außengewinde aufweisen, um das Fixierelement aufzunehmen. Dies bietet den Vorteil, dass das Fixierelement an dem Gewindeabschnitt fixiert werden kann.

Die Verbindungseinheit kann gemäß einer Ausführungsform ein Fixierelement aufweisen, das an dem Gewindeabschnitt befestigt ist, insbesondere wobei das Fixierelement als Mutter ausgeformt ist. Das Fixierelement bietet den Vorteil, dass das Verbindungselement formschlüssig mit dem Rohrabschnitt verbunden werden kann. Dabei kann das Fixierelement als Sechskantmutter ausgebildet sein. Dadurch entstehen geringe Produktionskosten sowie eine einfache Handhabung durch den Bediener.

Die Verbindungseinheit kann gemäß einer Ausführungsform ein Düsenelement aufweisen, das mit dem Verbindungselement gekoppelt ist oder koppelbar ist. Das Düsenelement kann insbesondere ausgebildet sein, um ein durch die Durchgangsöffnung strömendes Fluid von der proximalen Seite des Düsenelements, an der das Düsenelement am Verbindungselement gekoppelt ist, zu seiner distalen Seite zu leiten und über die dort angeordnete Düsenaustrittsöffnung des Düsenelements in das Reinigungsgerät abzugeben. Hierdurch kann Reinigungsgut gezielt mit dem aus dem Düsenaustrittsöffnung austretendem Fluid beaufschlagt werden. Dies gewährleistet eine optimale Reinigung von beispielsweise medizinischem Reinigungsgut, insbesondere etwa von Laborgläsern. Ein medizinisches Reinigungsgut, insbesondere etwa ein Laborglas, wird dabei für den Reinigungsprozess zumindest teilweise über das Düsenelement gestülpt, wodurch eine gute Innenreinigung des medizinischen Reinigungsguts bewirkt werden kann.

Das Düsenelement kann gemäß einer Ausführungsform mittels eines Innengewindes des Gewindeabschnittes mit dem Verbindungselement gekoppelt sein. Dies gewährleistet eine einfache Handhabung durch den Bediener. Das Düsenelement kann ohne großen Aufwand montiert und demontiert werden.

Alternativ kann das Düsenelement aber auch mit dem Verbindungselement verschweißt sein.

Das Rohr kann gemäß einer Ausführungsform im Rohrabschnitt im Querschnitt mehreckig, insbesondere viereckig und/oder zumindest im Bereich der Durchgangsöffnung eben ausgeformt sein. Dies gewährleistete eine einfache und kostengünstige Herstellung.

Das Reinigungsgerät kann gemäß einer Ausführungsform eine Verbindungseinheit aufweisen. Unter einem Reinigungsgerät kann vorliegend ein Gerät zur Reinigung und/oder Desinfektion verstanden werden. Dies bietet den Vorteil, dass beispielsweise medizinisches Reinigungsgut optimal von innen gereinigt werden kann. Die Verbindungseinheit kann vom Bediener schnell und ohne großen Zeitaufwand in dem Reinigungsgerät montiert und demontiert werden.

Besonders günstig ist es gemäß einer Ausführungsform auch, ein Verfahren zum Herstellen einer Verbindungseinheit vorzusehen, wobei das Verfahren die folgenden Schritte umfasst: Bereitstellen eines Rohres mit einem Rohrabschnitt und einem Verbindungselement mit einem Gewindeabschnitt und einem Kontaktabschnitt, wobei der Rohrabschnitt eine Durchgangsöffnung aufweist, die ausgebildet ist, um zumindest einen Teil eines Verbindungselementes aufzunehmen; und wobei der Gewindeabschnitt ausgebildet ist, um ein Fixierelement aufzunehmen, und formschlüssiges Verbinden des zumindest einen Teils des Verbindungselementes im Rohrabschnitt, um die Verbindungseinheit herzustellen.

Auch wenn der beschriebene Ansatz anhand eines Reinigungsgerätes beschrieben wird, kann die hier beschriebene Verbindungseinheit und das hier beschriebene Verfahren entsprechend im Zusammenhang mit einem gewerblichen oder professionellen Gerät, beispielsweise einem medizinischen Gerät, wie einem Reinigungs- oder Desinfektionsgerät, einem Kleinsterilisator, einem Großraumdesinfektor oder einer Container-Waschanlage eingesetzt werden.

Ein Ausführungsbeispiel der Erfindung ist in den Zeichnungen rein schematisch dargestellt und wird nachfolgend näher beschrieben. Es zeigt
- Figur 1: eine Explosionsdarstellung eines Ausführungsbeispiels einer Verbindungseinheit;
- Figur 2: eine Explosionsdarstellung eines Ausführungsbeispiels einer Verbindungseinheit;
- Figur 3: eine schematische Darstellung eines Ausführungsbeispiels einer Verbindungseinheit;
- Figur 4: eine Unteransicht eines Innenraumes eines Ausführungsbeispiels einer Verbindungseinheit;
- Figur 5: einen Schnitt durch eine Seitenansicht eines Ausführungsbeispiels einer Verbindungseinheit;
- Figur 6: eine schematische Darstellung eines Ausführungsbeispiels eines Korbes zum Reinigen von medizinischem Reinigungsgut;
- Figur 7: eine schematische Darstellung eines Ausführungsbeispiels eines Korbes zum Reinigen von medizinischem Reinigungsgut;
- Figur 8: eine schematische Darstellung eines Ausführungsbeispiels eines Korbes zum Reinigen von medizinischem Reinigungsgut;
- Figur 9: eine schematische Darstellung eines Ausführungsbeispiels eines Düsenelementes an einem Verbindungselement;
- Figur 10: eine schematische Darstellung eines Ausführungsbeispiels eines Düsenelementes an einem Verbindungselement;
- Figur 11: eine schematische Darstellung eines Ausführungsbeispiels eines Düsenelementes an einem Verbindungselement;

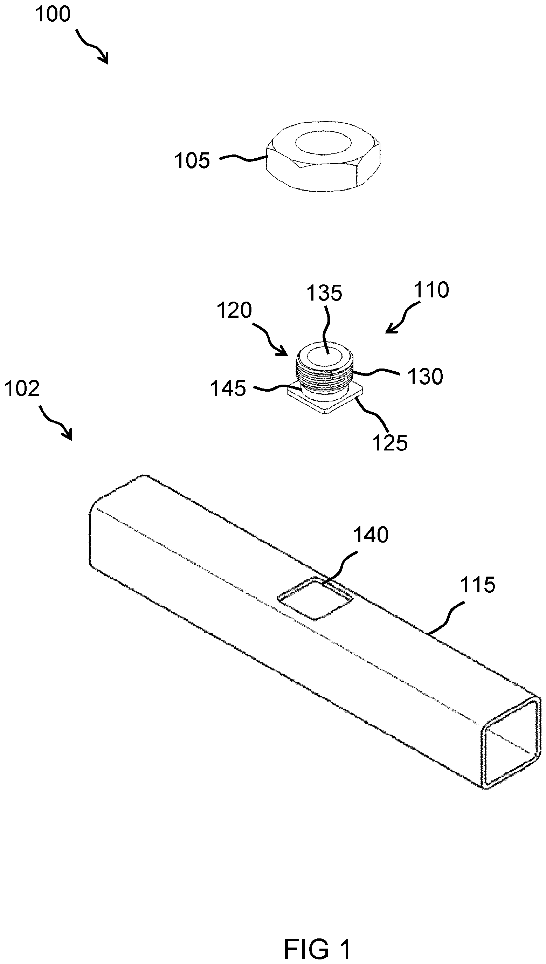

Figur 1 zeigt eine Explosionsdarstellung eines Ausführungsbeispiels einer Verbindungseinheit 100. Die Verbindungseinheit 100 lässt sich beispielhaft in einem Reinigungsgerät einsetzen. Bei dem Reinigungsgerät kann es sich um ein Reinigungsgerät zum Reinigen von medizinischem Reinigungsgut handeln. Die Verbindungseinheit 100 besteht aus einem Fixierelement 105, einem Verbindungselement 110 und einem Rohr 102 mit einem Rohrabschnitt 115. Das Verbindungselement 110 weist einen Gewindeabschnitt 120 und einen Kontaktabschnitt 125 auf. Der Gewindeabschnitt 120 umfasst hier ein Außengewinde 130 und ein Innengewinde 135 und ist ausgebildet, um das Fixierelement 105 aufzunehmen.

Das Außengewinde 130 nimmt dabei das Fixierelement 105 auf, wobei das Fixierelement 105 beispielhaft als Mutter ausgeformt ist. Das Verbindungselement 110 weist einen Halsabschnitt 145 zwischen dem Kontaktabschnitt 125 und dem Gewindeabschnitt 120 auf, wobei der Halsabschnitt 145 optional eine Länge aufweist, die mindestens einer Dicke einer Wand des Rohres 102 im Rohrabschnitt 115 entspricht. Der Halsabschnitt 145 weist beispielhaft zudem einen Querschnitt auf, der höchstens einer Breite des Kontaktabschnittes 125 entspricht. Der Kontaktabschnitt 125 ist ausgebildet, um den zumindest einen Teil des Verbindungselementes 110 im Rohrabschnitt 115 formschlüssig mit dem Rohr 102 zu verbinden. Um den zumindest einen Teil des Verbindungselementes 100 im Rohrabschnitt 115 aufzunehmen, weist der Rohrabschnitt 115 eine Durchgangsöffnung 140 auf. Der Kontaktabschnitt 125 weist beispielhaft im Querschnitt eine Form auf, die einer Form der Durchgangsöffnung 140 entspricht, um den zumindest einen Teil des Verbindungselementes 110 durch die Durchgangsöffnung 140 aufzunehmen. Die Durchgangsöffnung 140 im Rohrabschnitt 115 und der Kontaktabschnitt 125 des Verbindungselementes 110 sind beispielhaft viereckig ausgeformt. Optional können sie auch mehreckig, insbesondere dreieckig ausgeformt sein. Der Kontaktabschnitt 125 ist so ausgebildet, dass er in dem Rohrabschnitt 115 verkanten kann, da die größte Quererstreckung vom Kontaktabschnitt 125 größer ist als die Breite von der Durchgangsöffnung 140 vom Rohrabschnitt 115. Das Rohr 102 im Rohrabschnitt 115 ist mehreckig ausgeformt. Optional kann es auch nur im Bereich der Durchgangsöffnung 140 mehreckig oder eben, d.h. insbesondere eine ebene Oberfläche aufweisend, ausgeformt sein.

Durch die Erfindung werden die Fertigungskosten gesenkt. Außerdem wird eine einfachere Möglichkeit geschaffen einen Gewindeanschluss mit einem Rohr 102 zu erstellen, der keinen Schweißvorgang erfordert. Die Flexibilität wird sowohl in der Fertigung als auch beim Kunden erhöht, indem die entsprechende Gewindeverbindung im letzten Fertigungsschritt oder auch erst beim Kunden montiert wird. Im Fehlerfall wird der Gewindeabschnitt 120, der auch als Gewinde bezeichnet werden kann, beim Bediener einfach ausgetauscht, ohne dass z. B. ein ganzer Korb ausgetauscht werden braucht. Für die Erfindung werden hier zwei Bauteile verwendet, ein Fixierelement 105 und ein Verbindungselement 110. Das Verbindungselement 110, das auch als erstes Bauteil bezeichnet werden kann, wird beispielsweise aus einem Vierkant-Halbzeug hergestellt, bei dem durch Zerspannen ein Außengewinde 130 und Innengewinde 135 erstellt wird, wobei am Ende ein kurzes Stück vom Vierkant (1-1,5 mm) verbleibt. Das Fixierelement 105, das auch als zweites Bauteil bezeichnet werden kann, ist beispielsweise eine Standardmutter, die nicht vollständig auf das Außengewinde 130 des Verbindungselementes 110 geschraubt wird. Im Rohr 102, auf welchem der Gewindeabschnitt 120, der auch als Gewinde bezeichnet werden kann, montiert werden soll, wird nur eine viereckige Durchgangsöffnung 140, die auch als Öffnung bezeichnet werden kann, beispielsweise durch einen Laser, eingebracht. Das Fixierelement 105 und das Verbindungselement 110 können nun mit dem Kontaktabschnitt 125, der auch als Vierkantende bezeichnet werden kann, in diese Durchgangsöffnung 140 eingesetzt werden bis das Fixierelement 105, das auch als Sechskantmutter bezeichnet werden kann, auf dem Rohr 102 aufliegt und der Kontaktabschnitt 125 bis unter die Rohrwandung ragt. Wird nun das Fixierelement 105 weiter aufgeschraubt, dreht sich das Verbindungselement 110, bis der Kontaktabschnitt 125 an der Rohrwand anliegt und sich nicht weiter drehen kann. Das Fixierelement 105 kann dann vollständig angezogen werden und durch den verdrehten Kontaktabschnitt 125 kommt eine formschlüssige Verbindung zustande. In das Innengewinde 135 können alle bisher verwendeten Düsen/Adapter eingeschraubt werden.

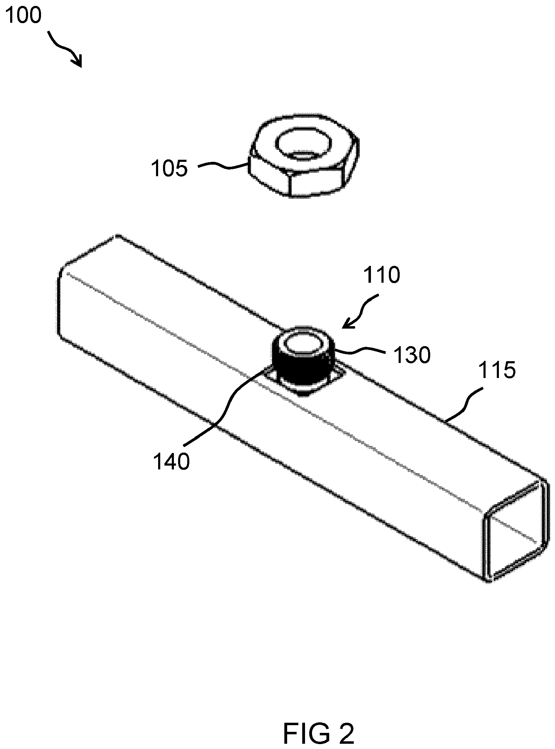

Figur 2 zeigt eine Explosionsdarstellung eines Ausführungsbeispiels einer Verbindungseinheit 100. Dabei kann es sich um die in Figur 1 beschriebene Verbindungseinheit 100 handeln. In Figur 2 befindet sich das Verbindungselement 110 in der Durchgangsöffnung 140 des Rohrabschnittes 115. Das Außengewinde 130 des Verbindungselementes 110 hat das Fixierelement 105 noch nicht aufgenommen.

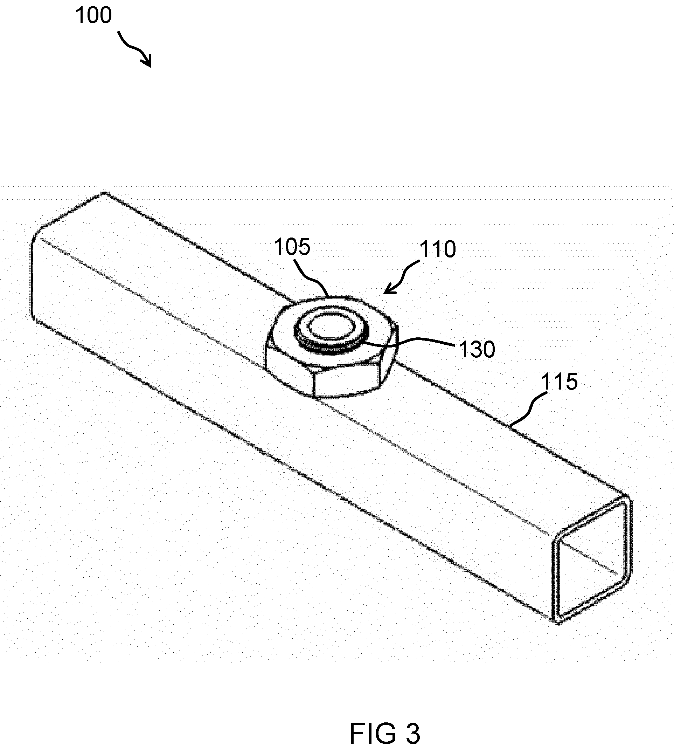

Figur 3 zeigt eine schematische Darstellung eines Ausführungsbeispiels einer Verbindungseinheit 100. Dabei kann es sich um die in Figur 1 und Figur 2 beschriebene Verbindungseinheit 100 handeln. In Figur 3 hat das Außengewinde 130 des Verbindungselementes 110 das Fixierelement 105 nun aufgenommen. Das Verbindungselement 110 befindet sich formschlüssig am Rohrabschnitt 115.

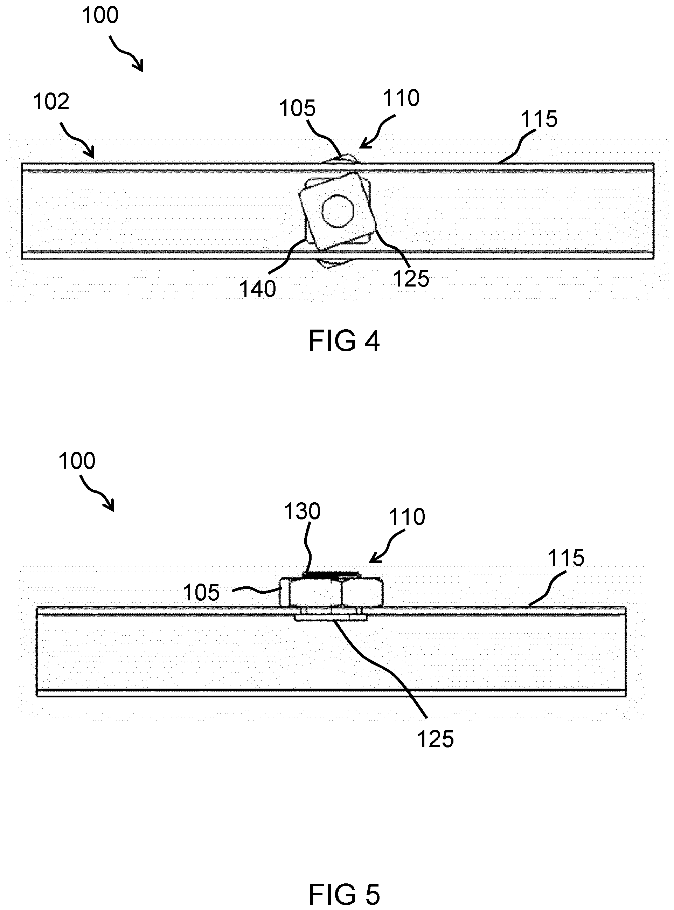

Figur 4 zeigt eine Unteransicht eines Innenraumes eines Ausführungsbeispiels einer Verbindungseinheit 100. Zu sehen ist das Verbindungselement 110 von unten. Der Kontaktabschnitt 125 ist in der Durchgangsöffnung 140 des Rohrabschnittes 115 verkantet. Somit ist eine formschlüssige Verbindung von dem Verbindungselement 110 mit dem Rohrabschnitt 115 des Rohres 102 gewährleistet. Um den Kontaktabschnitt 125 in der Durchgangsöffnung 140 des Rohrabschnittes 115 zu verkanten, wird das Fixierelement 105 nach dem Einsetzen des Verbindungselementes 110 in der Durchgangsöffnung 140 weiter aufgeschraubt. Der Kontaktabschnitt 125 dreht sich bis zum Anschlag im Rohr 102. Es ist keine weitere Rotation vom Kontaktabschnitt 125 möglich. Anschließend wird das Fixierelement 105 vollständig festgeschraubt.

Figur 5 zeigt einen Schnitt durch eine Seitenansicht eines Ausführungsbeispiels einer Verbindungseinheit 100. Dabei kann es sich um die in Figur 4 beschriebene Verbindungseinheit 100 handeln. Das Fixierelement 105 befindet sich am Außengewinde 130 des Verbindungselementes 110. Der Kontaktabschnitt 125 ist in der Durchgangsöffnung des Rohrabschnittes 115 verkantet. Das Fixierelement 105 ist vollständig festgeschraubt. Das Verbindungselement 110 ist formschlüssig in der Durchgangsöffnung montiert.

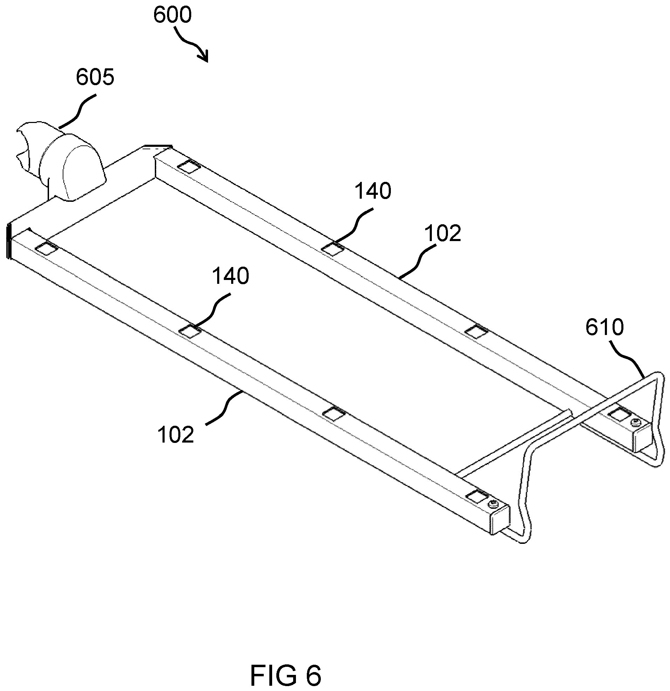

Figur 6 zeigt eine schematische Darstellung eines Ausführungsbeispiels eines Korbes oder eines Korbeinsatzes oder -modules zum Reinigen von medizinischem Reinigungsgut, insbesondere von Laborglas. Im Folgenden wird der Korb, der Korbeinsatz oder das Korbmodul vereinfacht als Korb 600 bezeichnet. Der Korb 600 ist ausgebildet, um entweder als separates Beladungselement in einem Reinigungsgerät oder in einem übergeordneten Beladungselement eines Reinigungsgeräts, welche eine Beladungsebene des Reinigungsgeräts bildet, eingehängt bzw. befestigt und angeschlossen zu werden. Dazu weist der Korb 600 an seinem einen Ende eine Kopplungseinheit 605 auf. Die Kopplungseinheit 605 wird an einer Rück- oder Seitenwand eines Reinigungsgerätes oder an einem übergeordneten Beladungselement des Reinigungsgeräts befestigt bzw. angeschlossen. Die Kopplungseinheit 605 weist eine Öffnung auf, durch die ein im Betrieb des Reinigungsgeräts von einem Auslassstutzen des Reinigungsgeräts bereitgestelltes Reinigungsfluid in die Rohre 102 gelangt. Die Rohre 102 verlaufen beispielsweise parallel zueinander. Der Korb 600 weist vorzugsweise mehrere gemäß diesem Ausführungsbeispiel beispielsweise zwei Rohre 102 auf, die wiederum eine Anzahl von Durchgangsöffnungen 140 aufweisen. In Figur 6 weist jedes Rohr 102 z.B. vier Durchgangsöffnungen 140 auf. An dem anderen Ende des Korbes 600 befindet sich ein Griff 610. Der Griff 610 dient dazu, dass der Bediener den Korb 600 aus dem Reinigungsgerät oder dem übergeordneten Beladungselement ziehen oder entnehmen kann bzw. den Korb 600 in das Reinigungsgerät einschieben kann.

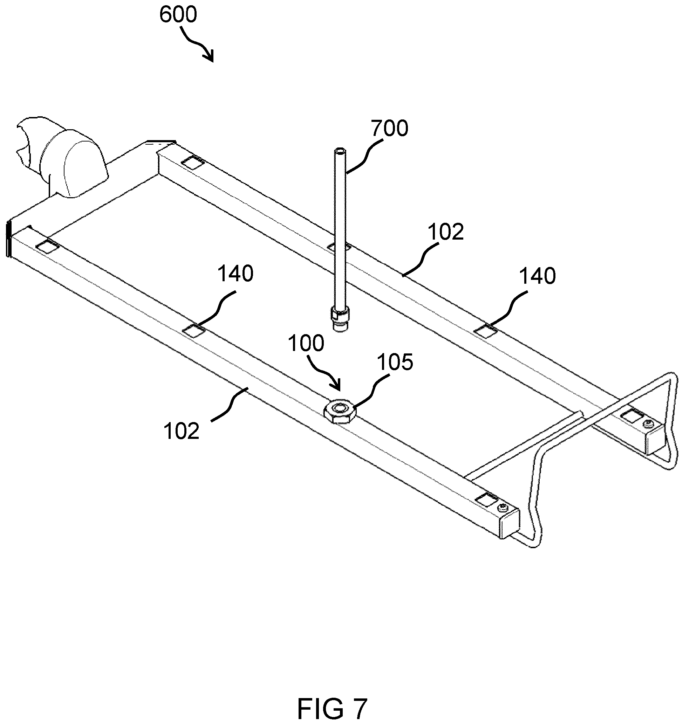

Figur 7 zeigt eine schematische Darstellung eines Ausführungsbeispiels eines Korbes 600 zum Reinigen von medizinischem Reinigungsgut, insbesondere von Laborglas. Dabei kann es sich um den in Figur 6 beschriebenen Korb 600 handeln. In Figur 7 befindet sich das Verbindungselement in der Durchgangsöffnung 140 des Rohres 102. Das Fixierelement 105 befindet sich am Verbindungselement. Ein Düsenelement 700 ist explosionsartig über der Verbindungseinheit 100 angeordnet. Der Bediener schraubt das Düsenelement 700 in das Innengewinde des Verbindungselementes, um es formschlüssig an die Verbindungseinheit 100 zu koppeln.

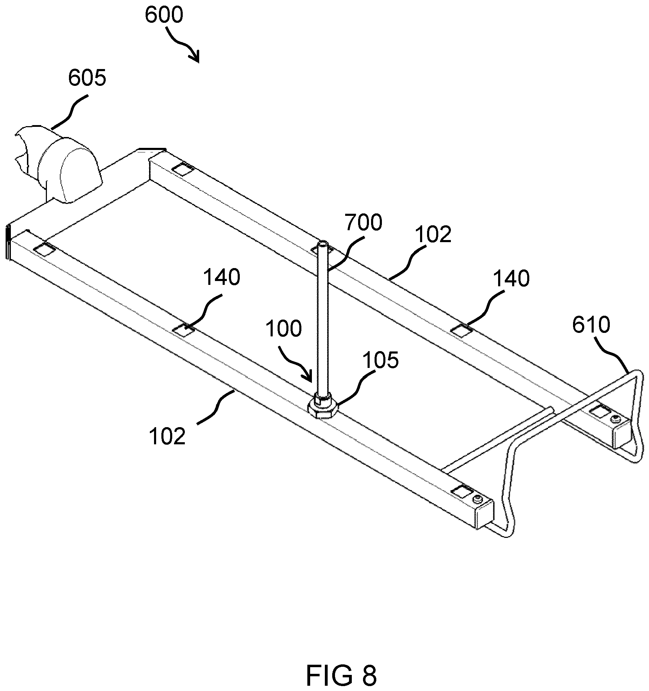

Figur 8 zeigt eine schematische Darstellung eines Ausführungsbeispiels eines Korbes 600 zum Reinigen von medizinischem Reinigungsgut, insbesondere von Laborglas. Dabei kann es sich um den in Figur 6 und in Figur 7 beschriebenen Korb 600 handeln. In Figur 8 ist das Düsenelement 700 an der Verbindungseinheit 100 angekoppelt. Das Düsenelement 700 ist mittels eines Innengewindes des Gewindeabschnittes mit dem Verbindungselement gekoppelt. Das Reinigungsfluid gelangt von der Öffnung der Kopplungseinheit 605 in die Rohre 102. Durch die Durchgangsöffnungen 140 in den Rohren 102 gelangt das Reinigungsfluid anschließend in das Düsenelement 700. Über das Düsenelement 700 kann beispielsweise ein Laborglas gestülpt werden, das auf diese Weise dann gereinigt und desinfiziert wird. Vorzugsweise ist der Korb ausgebildet, um mehrere, beispielsweise wie in Figur 8 gezeigt insgesamt acht Düsenelemente 700 aufzunehmen. Der Bediener hat in Figur 8 das Düsenelement 700 in das Innengewinde des Verbindungselementes geschraubt, um es formschlüssig an die Verbindungseinheit 100 zu koppeln. Der Bediener kann nun den Korb 600 in diesem Beispiel mit insgesamt bis zu acht Düsenelementen 700 versehen, indem er die Düsenelemente 700 in das Innengewinde des Verbindungselementes verschraubt. Anschließend kann er das zu reinigende und zu desinfizierende Reinigungsgut, beispielsweise Laborgläser, auf die Düsenelemente 700 stülpen. Der Korb 600 kann nun vom Bediener mithilfe des Griffes 610 in das Reinigungsgerät geschoben oder in ein übergeordnetes Beladungselement, welches in das Reinigungsgerät eingeschoben werden kann, eingebracht werden. Beim Hineinschieben des Korbes 600 oder dem übergeordneten Beladungselement in das Reinigungsgerät wird die Kopplungseinheit 605 automatisch an die Wasserzufuhreinrichtung im Reinigungsgerät gekoppelt. Nach dem Starten des Reinigungsgerätes wird das Reinigungsfluid mittels einer Pumpe in die Öffnung der Kopplungseinheit 605 gepumpt. Von dort gelangt das Reinigungsfluid in die Rohre 102 und durch die Durchgangsöffnungen 140 in die Düsenelemente 700. Am oberen Ende der Düsenelemente 700 strömt das Reinigungsfluid heraus und reinigt bzw. desinfiziert das Reinigungsgut.

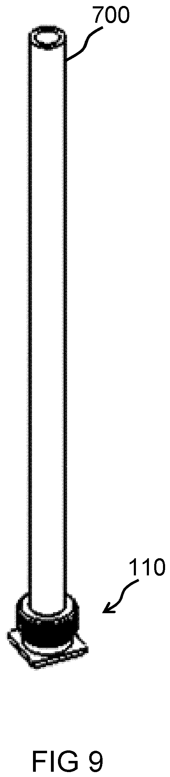

Figur 9 zeigt eine schematische Darstellung eines Ausführungsbeispiels eines Düsenelementes 700 an einem Verbindungselement 110. In Figur 9 ist eine Variante des Verbindungselementes 110 dargestellt. Bei dieser Variante kann das Verbindungselement 110, welches auch als das erste Bauteil bezeichnet werden kann, das bisherige Gewindeende an dem Düsenelement 700, das auch als Düse bezeichnet werden kann, ersetzen, indem das Innengewinde des Verbindungselements 110 durch eine Bohrung ersetzt wird, in welcher das Düsenelement 700, das auch als Düsenrohr bezeichnet werden kann, aufgenommen wird. So kann gegenüber der in den Figuren 1 bis 5, 7-8 gezeigten Ausführung ein Bauteil eingespart werden, allerdings könnten dann die bisher eingesetzten Düsenelemente 700 nicht weiter verwendet werden. Hier wird das Düsenelement 700 direkt mit dem neuen Verbindungselement 110 verschweißt und so das bisherige Gewindestück des Düsenelementes 700 eingespart. Somit wäre eine grundsätzliche Umstellung erforderlich.

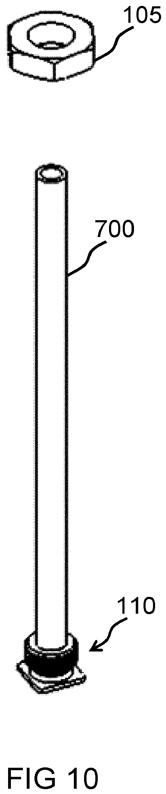

Figur 10 zeigt eine Darstellung eines Ausführungsbeispiels eines Düsenelementes 700 an einem Verbindungselement 110. Dabei kann es sich um das in Figur 9 beschriebene Düsenelement 700 handeln. In Figur 10 ist das Fixierelement 105 explosionsartig über dem Düsenelement 700 dargestellt.

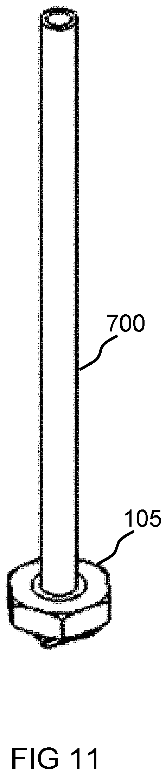

Figur 11 zeigt eine Darstellung eines Ausführungsbeispiels eines Düsenelementes 700 an einem Verbindungselement 110. Dabei kann es sich um das in Figur 9 und in Figur 10 beschriebene Düsenelement 700 handeln. Das Düsenelement 700 ist an dem Verbindungselement 110 angeschweißt und kann zusammen mit diesem mittels Fixierelement 105 an einem, hier nicht gezeigten Rohr 102 analog zu dem Ausführungsbeispiel der Figuren 1 bis 8 angebracht werden.

## Patentansprüche

1. Verbindungseinheit (100) zum Verbinden eines Verbindungselementes (110) mit einem Rohrabschnitt (115) für einen Einsatz in einem Reinigungsgerät, wobei die Verbindungseinheit (100) die folgenden Merkmale aufweist:
- ein Rohr (102) mit dem Rohrabschnitt (115), wobei der Rohrabschnitt (115) eine Durchgangsöffnung (140) aufweist, die ausgebildet ist, um zumindest einen Teil eines Verbindungselementes (110) aufzunehmen;
- das Verbindungselement (110) mit einem Gewindeabschnitt (120) und einem Kontaktabschnitt (125), wobei der Gewindeabschnitt (120) ausgebildet ist, um ein Fixierelement (105) aufzunehmen, und wobei der Kontaktabschnitt (125) ausgebildet ist, um den zumindest einen Teil des Verbindungselementes (110) im Rohrabschnitt (115) formschlüssig mit dem Rohr (102) zu verbinden.

2. Verbindungseinheit (100) gemäß Anspruch 1, wobei der Kontaktabschnitt im Querschnitt eine Form aufweist, die einer Form der Durchgangsöffnung (140) entspricht, um den zumindest einen Teil des Verbindungselementes (110) durch die Durchgangsöffnung (140) aufzunehmen.

3. Verbindungseinheit (100) gemäß Anspruch 2, wobei die Durchgangsöffnung (140) im Rohrabschnitt (115) und/oder der Kontaktabschnitt (125) mehreckig insbesondere im Wesentlichen dreieckig oder viereckig ausgeformt ist.

4. Verbindungseinheit (100) gemäß einem der vorangegangenen Ansprüche, wobei der Kontaktabschnitt (125) in dem Rohrabschnitt (115) verkantbar ausgebildet ist.

5. Verbindungseinheit (100) gemäß einem der vorangegangenen Ansprüche, wobei das Verbindungselement (110) einen Halsabschnitt (145) zwischen dem Kontaktabschnitt (125) und dem Gewindeabschnitt (120) aufweist, wobei der Halsabschnitt (145) eine Länge aufweist, die mindestens einer Dicke einer Wand des Rohres (102) im Rohrabschnitt (115) entspricht.

6. Verbindungseinheit (100) gemäß Anspruch 5, wobei der Halsabschnitt (145) einen Querschnitt aufweist, der höchstens einer Breite des Kontaktabschnittes (125) entspricht.

7. Verbindungseinheit (100) gemäß einem der vorangegangenen Ansprüche, wobei das Verbindungselement (110) im Gewindeabschnitt (120) ein Außengewinde (130) aufweist, um das Fixierelement (105) aufzunehmen.

8. Verbindungseinheit (100) gemäß Anspruch 7, mit einem Fixierelement (105), das an dem Gewindeabschnitt (120) befestigt ist, insbesondere wobei das Fixierelement (105) als Mutter ausgeformt ist.

9. Verbindungseinheit (100) gemäß einem der vorangegangenen Ansprüche, mit einem Düsenelement (700), das mit dem Verbindungselement (110) gekoppelt ist, insbesondere wobei das Düsenelement (700) ausgebildet ist, um ein aus der Durchgangsöffnung (140) strömendes Fluid zu leiten.

10. Verbindungseinheit (100) gemäß Anspruch 9, wobei das Düsenelement (700) mittels eines Innengewindes (135) des Gewindeabschnittes (120) mit dem Verbindungselement (110) gekoppelt ist.

11. Verbindungseinheit (100) gemäß einem der vorangegangenen Ansprüche, wobei das Rohr (102) im Rohrabschnitt (115) im Querschnitt mehreckig und/oder zumindest im Bereich der Durchgangsöffnung (140) eben ausgeformt ist.

12. Reinigungsgerät mit einer Verbindungseinheit (100) gemäß einem der vorangegangenen Ansprüche 1 bis 11.

13. Verfahren (1200) zum Herstellen einer Verbindungseinheit (100) gemäß einem der vorangegangen Ansprüche, wobei das Verfahren (1200) die folgenden Schritte (1205, 1210) aufweist:
- Bereitstellen (1205) eines Rohres (102) mit einem Rohrabschnitt (115) und einem Verbindungselement (110) mit einem Gewindeabschnitt (120) und einem Kontaktabschnitt (125), wobei der Rohrabschnitt (115) eine Durchgangsöffnung (140) aufweist, die ausgebildet ist, um zumindest einen Teil eines Verbindungselementes (110) aufzunehmen; und wobei der Gewindeabschnitt (120) ausgebildet ist, um ein Fixierelement (105) aufzunehmen, und
- formschlüssiges Verbinden (1210) des zumindest einen Teils des Verbindungselementes (110) im Rohrabschnitt (115), um die Verbindungseinheit (100) herzustellen.
